# EUROPÄISCHE PATENTSCHRIFT

(11) **EP 4 340 916 B1**
(45) Veröffentlichungstag und Bekanntmachung des Hinweises auf die Patenterteilung: **02.07.2025**
(21) Anmeldenummer: 22729079.8
(22) Anmeldetag: 11.05.2022
(51) Int. Cl.: A61M 16/04

(54) **GESCHLOSSENE INTUBATIONSSYSTEME MIT VERBESSERTEM ATEMWEGSZUGANG FÜR UNTERSUCHUNGSVORRICHTUNGEN**
CLOSED INTUBATION SYSTEMS HAVING IMPROVED AIRWAY ACCESS FOR EXAMINATION DEVICES
SYSTÈMES D'INTUBATION FERMÉS AYANT UN ACCÈS AUX VOIES RESPIRATOIRES AMÉLIORÉ POUR DES DISPOSITIFS D'EXAMEN

(30) Priorität: 19.05.2021 DE 102021113007
(43) Veröffentlichungstag der Anmeldung: 27.03.2024
(73) Patentinhaber: Universität zu Köln, 50931 Köln (DE)
(72) Erfinder: KÖHLER, Philipp, 50859 Köln (DE); CORNELY, Oliver Andreas, 50859 Köln (DE); KOCHANEK, Matthias, 51065 Köln (DE)
(74) Vertreter: Michalski Hüttermann & Partner Patentanwälte mbB
(86) Internationale Anmeldenummer: PCT/EP2022/062758
(87) Internationale Veröffentlichungsnummer: WO 2022/243128

(56) Entgegenhaltungen:
- US-A- 4 696 296
- US-A- 5 083 561
- US-A- 5 300 043
- US-A- 5 746 199
- US-A1- 2014 121 607
- US-A1- 2015 343 182

## Beschreibung

Die vorliegende Erfindung betrifft ein geschlossenes Intubationssystem mindestens umfassend einen für die Einführung in die Luftröhre eines Patienten geeigneten Endotrachealtubus, eine schlauchförmige Anbindung an eine Beatmungsvorrichtung und eine Adapteranordnung mit mindestens drei zylindrisch ausgestalteten Aufnahmen zur Herstellung einer gasdichten Kopplung zwischen Endotrachealtubus, der Anbindung der Beatmungsvorrichtung und mindestens einer weiteren Aufnahme, wobei die Adapteranordnung mindestens umfasst:
a) einen über die Anordnung der Aufnahmen T-förmig ausgestalteten Adaptergrundkörper mit mindestens einer Absperrvorrichtung, wobei die Absperrvorrichtung dazu eingerichtet ist den Luftweg zwischen der weiteren Aufnahme und den beiden Aufnahmen für die Beatmungsvorrichtung und den Endotrachealtubus gasdicht zu öffnen oder zu schließen,
b) einen an die weitere Aufnahme ankoppelbaren Adapteraufsatz, wobei der Adapteraufsatz über eine Dichtung gasdicht mit dem Adaptergrundkörper verbindbar und über Haltemittel an diesem mechanisch fixierbar ausgestaltet ist;
c) eine mit einem Ende am Adapteraufsatz gasdicht angeordnete elastische, schlauchförmige Hülle;
d) einem mit dem anderen Ende der elastischen, schlauchförmigen Hülle gasdicht verbundenen Aufsatz, wobei der Aufsatz einen Durchgang zur schlauchförmigen Hülle mit einer darin angeordneten Dichtvorrichtung umfasst, wobei die Dichtvorrichtung dazu eingerichtet ist zylindrisch ausgestaltete Vorrichtungen aufzunehmen und diese durch den Aufsatz, die schlauchförmige Hülle, den Adapteraufsatz, den Adaptergrundkörper und den Endotrachealtubus verschiebbar und gasdicht zu führen.

Die im Gesundheitssystem tätigen Mitarbeiter sind heutzutage einer steigenden Anzahl an persönlichen Belastungen ausgesetzt. Dies gilt in hohem Maße für das in Kliniken tätige Personal, wobei hier die Pflegekräfte mit direktem Patientenkontakt und insbesondere das intensivmedizinische Personal zu nennen sind. Für letztere Gruppen hat die stetige Verdichtung der Arbeitsleistung und der generelle Mangel an Pflegekräften in den letzten Jahren zu einem besonderen Anstieg des eigenen Risikopotentials geführt, welches durch die über SARS-CoV-2 ausgelöste Pandemielage, mit deutlich steigenden intensivmedizinischen Fallzahlen und deutlich erhöhter Patienteninfektiosität, zusätzlich verschärft wurde. Viele künstlich beatmete COVID-19-Patienten leiden an einem "acute respiratory distress syndrome" (ARDS), dessen Behandlung eine positiv druckkontrollierte Beatmung mit einem positiven endexspiratorischen Druck (PEEP) und Einsatz einer befeuchteten, temperaturkontrollierten Beatmungsluft erfordert. In der intensivmedizinischen Behandlung kann es aber immer wieder vorkommen, dass aufgrund lebensbedrohender Komplikationen, wie beispielsweise schwerwiegender Lungenblutungen (>200ml/24h), Fremdkörpern in der Lunge, Atelektasen oder ähnlichem, eine umgehende Bronchoskopie oder eine bronchoalveoläre Lavage (BAL) zur Behandlung und Probengewinnung indiziert ist. Die Nofallmaßnahmen führen in der Regel dazu, dass der geschlossene Beatmungskreislauf unter Abgabe von Aerosolen und Tröpfchen an die Umgebungsluft geöffnet werden muss, welches für das unmittelbar involvierte medizinische Personal mit einem erheblichen Expositionsrisiko gegenüber Infektionserkrankungen (SARS-CoV-2, MERS, Influenza, Tuberkulose etc.) einhergeht. Aufgrund des oben beschriebenen Risikos werden von COVID-19 Patienten Proben hilfsweise aus dem oberen Atemwegstrakt gewonnen, was dazu führen kann, dass Sekundärinfektionen gar nicht oder zu spät diagnostiziert werden. Insofern wären technische Hilfsmittel wünschenswert, welche eine sichere und einfache Behandlung oder Beprobung der Lunge auch hochinfektiöser Patienten ohne Gefahr für das behandelnde Personal ermöglichen.

Auch in der Patentliteratur finden sich die unterschiedlichsten Ansätze zur Ausgestaltung geschlossener Intubationssysteme sowie mit diesen kompatibler Bronchoskope und Absaugkatheter.

So beschreibt beispielsweise die US 4,850,350 ein geschlossenes System mit einer kombinierten medizinisch-chirurgischen Absaug- und Beatmungsschlauchvorrichtungen umfassend (1) einen Absaugkatheter, (2) ein Absaugsteuerventil, das am distalen Ende des Systems positioniert ist, (3) eine Mehrfachanschlusskopplungseinheit, durch die der Katheter verläuft zur Aspiration eines Patienten zugeführt werden, (4) eine flexible röhrenförmige Hülle, die eine Sterilitätsschutzhülle für den Katheter bereitstellt, der an seinem distalen Ende am proximalen Ende der Kopplungseinheit befestigt ist, und (5) ein Hüllentlastungsventil. Die Kopplungseinheiten weist zwei getrennte Elemente auf, von denen eines axial in das andere eingepasst ist, so dass sie sich relativ zueinander um ihre Längsachsen drehen können, um den Zugang des Katheters von der Hülle durch die Kopplungseinheit in den Patienten zu schließen und zu öffnen. Die Saugsteuerventile haben erste und zweite im wesentlichen identische Teile, die entlang einer Längsseite in gleitendem Eingriff miteinander positioniert sind, um sich zwischen einer offenen und einer geschlossenen Position der Ventile zu bewegen. Die Mantelentlastungsventile verhindert, dass Luft in der Hülle während der Verwendung der Baugruppe verunreinigt wird.

Die US 2010/0154799 A1 beschreibt eine Atemwegszugang-Baugruppe, umfassend: ein bewegbares Anschlussstück mit wenigstens einer Öffnung, wobei die Öffnung in Kommunikation mit einem künstlichen Luftweg eines Patienten steht; eine geschlossene Saugkatheter-Baugruppe, umfassend wenigstens ein Verbindungsende mit einer dadurch gebildeten Öffnung, einen Saugkatheter, und eine über dem Saugkatheter positionierte Hülle; einen Shuttle, welcher zwischen der Öffnung im Anschlussstück und der Öffnung im Verbindungsende der geschlossenen Saugkatheter-Baugruppe bewegbar ist, wobei der Shuttle eine dadurch gebildete Öffnung aufweist und konfiguriert ist um sich in eine gesperrte und eine entsperrte Position relativ zum Anschlussstück zu bewegen; ein Betätigungselement zum Bewegen des Shuttle in die gesperrte Position und die entsperrte Position; und eine Klappe, welche zu dem Shuttle und der geschlossenen Saugkatheter-Baugruppe benachbart positioniert ist, wobei die Klappe in eine geöffnete Position bewegbar ist, wobei Durchtritt des Saugkatheters in einen künstlichen Luftweg ermöglicht ist, wenn der Shuttle in einer gesperrten Position ist, und die Klappe in eine geschlossene Position bewegbar ist, wobei Durchtritt des Saugkatheters durch den Shuttle verhindert ist, wenn der Shuttle in einer entsperrten Position ist.

Die Möglichkeiten und die Wichtigkeit zum Schutz der Intensivmediziner vor ungewollten Infektionen durch den Austritt infektiöser Partikel während der Beatmung von COVID-19-Pateienten wurde beispielsweise durch Koehler P., Cornely O.A., Kochanek M., "Bronchoscopy safety precautions for diagnosing COVID-19 associated pulmonary aspergillosis - a simulation study" in Mycoses, 2020 gezeigt.

Des Weiteren zeigt die US 2014/121607 A1 ein umschaltbares Gerät für einen geschlossenen Absaugkatheter, welches einen Gerätekörper und ein Zweiwege-Umschaltventil umfasst. Der Gerätekörper besteht aus einer Ventilhülse, einem Patientenschlauchadapter, einem seitlichen Anschluss und einem Katheteradapter. In der Ventilhülse ist ein Hohlraum ausgebildet, der eine entsprechende Drehung des Zweiwege-Umschaltventils im Verhältnis zum Gerätekörper ermöglicht. Der Katheteradapter und der Patientenschlauchadapter sind an gegenüberliegenden Enden der Ventilhülse ausgebildet und können dadurch einen ungehinderten Weg zwischen ihnen herstellen. Der Seitenanschluss ist schräg geformt und erstreckt sich vom Patientenschlauchadapter aus, um einen ungehinderten Durchfluss zwischen ihnen zu gewährleisten. An einem Ende des Patientenschlauchadapters ist eine Nut als um 360 Grad frei drehbares Kupplungselement ausgebildet.

Derartige aus dem Stand der Technik bekannte Lösungen können noch weiteres Verbesserungspotential bieten, insbesondere hinsichtlich der Eignung zur Reduzierung der Exposition der Umgebungsluft in intensivmedizinischen Eingriffen unter geschlossenem Beatmungskreislauf.

Es ist daher die Aufgabe der vorliegenden Erfindung, die aus dem Stand der Technik bekannten Nachteile zumindest teilweise zu überwinden. Es ist insbesondere die Aufgabe der vorliegenden Erfindung ein geschlossenes Intubationssystem bereitzustellen, welches eine sichere Beatmung eines Pateinten und eine einfache Durchführung intensivmedizinischer Untersuchungen, ohne ungewollte Freisetzung von Aerosolpartikeln oder Tröpfchen in die Umgebungsluft, ermöglicht.

Die Lösung der Aufgabe erfolgt durch die Merkmale des auf die erfindungsgemäße Vorrichtung gerichteten unabhängigen Anspruchs. Bevorzugte Ausgestaltungen der Erfindung sind in den Unteransprüchen, in der Beschreibung oder den Figuren angegeben, wobei weitere in den Unteransprüchen oder in der Beschreibung oder den Figuren beschriebene oder gezeigte Merkmale einzeln oder in einer beliebigen Kombination einen Gegenstand der Erfindung darstellen können, solange sich aus dem Kontext nicht eindeutig das Gegenteil ergibt.

Erfindungsgemäß ist demzufolge ein geschlossenes Intubationssystem mindestens umfassend einen für die Einführung in die Luftröhre eines Patienten geeigneten Endotrachealtubus, eine schlauchförmige Anbindung an eine Beatmungsvorrichtung und eine Adapteranordnung mit mindestens drei zylindrisch ausgestalteten Aufnahmen zur Herstellung einer gasdichten Kopplung zwischen Endotrachealtubus, der Anbindung der Beatmungsvorrichtung und mindestens einer weiteren Aufnahme, wobei die Adapteranordnung mindestens umfasst:
a) einen über die Anordnung der Aufnahmen T-förmig ausgestalteten Adaptergrundkörper mit mindestens einer Absperrvorrichtung, wobei die Absperrvorrichtung dazu eingerichtet ist den Luftweg zwischen der weiteren Aufnahme und den beiden Aufnahmen für die Beatmungsvorrichtung und den Endotrachealtubus gasdicht zu öffnen oder zu schließen,
b) einen an die weitere Aufnahme ankoppelbaren Adapteraufsatz, wobei der Adapteraufsatz über eine Dichtung gasdicht mit dem Adaptergrundkörper verbindbar und über Haltemittel an diesem mechanisch fixierbar ausgestaltet ist;
c) eine mit einem Ende am Adapteraufsatz gasdicht angeordnete elastische, schlauchförmige Hülle;
d) einem mit dem anderen Ende der elastischen, schlauchförmigen Hülle gasdicht verbundenen Aufsatz, wobei der Aufsatz einen Durchgang zur schlauchförmigen Hülle mit einer darin angeordneten Dichtvorrichtung umfasst, wobei die Dichtvorrichtung dazu eingerichtet ist zylindrisch ausgestaltete Vorrichtungen aufzunehmen und diese durch den Aufsatz, die schlauchförmige Hülle, den Adapteraufsatz, den Adaptergrundkörper und den Endotrachealtubus verschiebbar und gasdicht zu führen.

Überraschenderweise hat sich gezeigt, dass mittels des geschlossenen Intubationssystems mit oben angegebenen Adapteraufbau eine einfache, schnelle und sichere Behandlung und Beprobung der Atemwege auch hochinfektiöser Patienten gewährleistet werden kann, ohne das Tröpfchen oder Aerosole aus dem geschlossenen Kreislauf an die Umgebungsluft entweichen können. Möglicherweise freigesetzte Teilchen werden sicher in der Adapteranordnung oder in der schlauchförmigen Hülle eingeschlossen und ein Einführen und Handling einer Untersuchungsvorrichtung, beispielsweise ein Bronchoskop, zum Untersuchungsort in der Lunge des Patienten wird nur in vernachlässigbarer Weise eingeschränkt. Auf diese Art und Weise wird eine Exposition der Umgebung und der behandelnden Personen durch potentiell infektiöse Teilchen sicher verhindert. Neben der Kontaminationssicherheit wird durch die Adapteranordnung auch eine schnelle Bedienbarkeit gewährleistet, welche insbesondere bei notfallmedizinischen, invasiven Maßnahmen von großer Wichtigkeit ist. Zudem kann durch den erfindungsgemäßen Aufbau, entgegen den Eigenschaften der aus dem Stand der Technik bekannten Aufbauten, eine sterile Handhabung von Bronchoskopen oder anderen Untersuchungsinstrumenten ermöglicht werden. Die Adapteranordnung ist zudem so flexibel, dass eine Reihe unterschiedlicher Maßnahmen, wie beispielsweise eine Bronchoskopie oder eine Lungenspülung, durchgeführt werden können. Über die spezielle Ausgestaltung der Anordnung ist zudem auch ein schnelles und sicheres Ausbringen von Proben und deren Isolierung sichergestellt. Dies kann neben der Ausführung einer sicheren Behandlung und der Arbeitssicherheit als solche auch den weiteren Untersuchungsfortgang vereinfachen.

Das hier betrachtete System ist ein geschlossenes Intubationssystem. Eine endotracheale Intubation wird im Allgemeinen dann angewendet, wenn Patienten eine mechanische Unterstützung der Atmung bedürfen. Eine weitere Anwendungsmöglichkeit besteht darin, dass bei fehlenden Schutzreflexen ein gesicherter Beatmungsweg geschaffen werden soll. Die Intubation verhindert die Verlegung des oberen Atemwegs und bietet einen Schutz vor Aspiration. Bei der Intubation wird ein Cuff (Blockmanschette, kleiner Ballon am Ende des Endotrachealtubus) in Tubuslage aufgeblasen und dichtet so die Luftröhre ab. Durch die Abdichtung können Mageninhalt, Blut oder Fremdkörper nicht oder nur in geringer Menge in die Lunge gelangen. In der endotrachealen Intubation wird ein Endotrachealtubus durch den Mund (orotracheal), Nase (nasotracheal) oder ein Tracheostoma in die Luftröhre (Trachea) eingebracht. Die Intubation gilt als Standardmethode der Atemwegssicherung und wird in der Anästhesie, Intensiv- und Notfallmedizin, narkotisierten oder bewusstlosen Patienten oder generell in Fällen akuter Störungen der Atmung eingesetzt. Der eingeführte Tubus kann ein einfacher oder Doppellumentubus sein, welcher die seitengetrennte Belüftung der Lungenflügel ermöglicht. Das System ist in den Fällen ein geschlossenes System, in denen während der Zwangsbeatmung abgesehen vom Beatmungsgerät durch das Intubationssystem kein signifikanter Luftaustausch mit der ungeschützten Umgebungsluft erfolgt.

Als Komponenten umfasst das Intubationssystem einen für die Einführung in die Luftröhre eines Patienten geeigneten Endotrachealtubus und eine schlauchförmige Anbindung an eine Beatmungsvorrichtung. Das Intubationssystem umfasst also mindestens eine Anbindung an eine Quelle für die Luft zur Beatmung des Patienten. Diese Vorrichtung kann neben dem notwendigen Druckerzeuger auch noch weitere Komponenten, beispielsweise einen Befeuchter und eine Temperiereinheit für die Atemluft aufweisen. Die Beatmungsvorrichtung weist des Weiteren eine geeignete Verbindung auf, mittels derer die Atemluft aus der Beatmungsvorrichtung zum Patienten geleitet wird. Dies kann über eine schlauchartige oder schlauchförmige Verbindung erfolgen, welche sowohl die Zu- wie auch die Abfuhr der Atemluft unmittelbar oder mittelbar zum Endotrachealtubus gewährleistet. Als weitere Komponente umfasst das System einen Endotrachealtubus, welcher in die Lunge des Patienten eingeführt werden kann. Es können unterschiedlich ausgestaltete Endotrachealtuben im Verbund mit den anderen Komponenten des Systems verwendet werden.

Das Intubationssystem umfasst zudem eine Adapteranordnung mit mindestens drei zylindrisch ausgestalteten Aufnahmen zur Herstellung einer gasdichten Kopplung zwischen Endotrachealtubus, der Anbindung der Beatmungsvorrichtung und mindestens einer weiteren Aufnahme. Als mechanisches und gasdichtes Verbindungs- oder Kupplungsstück dient die Adapteranordnung, in welcher die Zuleitung von der Beatmungsvorrichtung und die Anbindung des Endotrachealtubus zusammengeführt wird. Aufgrund der schlauchförmigen Ausgestaltung des Endotrachealtubus und der üblichen Anbindung der Beatmungsvorrichtung als Schlauchanbindung weist die Adapteranordnung also zylindrische Aufnahmen auf, an welchen die beiden Schlauchenden gasdicht fixiert werden können. Die mechanische Anbindung der Schläuche an die Aufnahmen kann über den Fachmann bekannte Anbindungsarten, beispielsweise durch mechanische Steck- oder Schraubverbindungen, erfolgen. Es ist aber auch möglich, dass die Aufnahmen jeweils unabhängig voneinander als Kupplungsstücke ausgestaltet sind, welche mit einem oder mehreren Dichtmitteln eine einfache Steckverbindung zwischen Adapteranordnung und Schläuchen ermöglicht. Neben den Aufnahmen in Richtung der Beatmungsvorrichtung und in Richtung des Endotrachealtubus weist die Adapteranordnung noch mindestens eine weitere Anschlussmöglichkeit auf, welche von den weiteren beiden Aufnahmen mechanisch unabhängig ist. Nicht erfindungsgemäß wäre also das Vorliegen nur zweier Ansatzstücke oder Aufnahmen, wobei der Luftweg zwischen einem oder mehreren der Komponenten Beatmungsvorrichtung, Endotrachealtubus und weiterem Anschluss vor dem Eingang in die Adapteranordnung zumindest teilweise zusammengeführt wird.

Die Adapteranordnung umfasst mindestens a) einen über die Anordnung der Aufnahmen T-förmig ausgestalteten Adaptergrundkörper mit mindestens einer Absperrvorrichtung, wobei die Absperrvorrichtung dazu eingerichtet ist den Luftweg zwischen der weiteren Aufnahme und den beiden Aufnahmen für die Beatmungsvorrichtung und den Endotrachealtubus gasdicht zu öffnen oder zu schließen. Der Luftweg über die weitere Aufnahme lässt sich gegenüber den Aufnahmen oder Anbindungen des Endotrachealtubus und der Beatmungsvorrichtung entkoppeln, sodass zwischen der weiteren Aufnahme und den beiden anderen Aufnahmen kein Luftfluss stattfindet. Die Absperrvorrichtung beeinflusst oder behindert dabei nicht den Luftdurchfluss von der Beatmungsvorrichtung zum Endotrachealtubus. Die Absperrvorrichtung kann dabei in der Form eines mechanisch oder elektrisch betätigbaren Ventils oder Verschlusses ausgestaltet sein. Es kann sich aber auch um eine oder mehrere reversibel schließ- und öffenbare Membranen handeln, sofern die Absperrvorrichtung einen Druckunterschied von beispielsweise mehr als 100 Pa, des Weiteren mehr als 500 Pa und des Weiteren bevorzugt mehr als 2,5 kPa zwischen den unterschiedlichen Beatmungsphasen der Zwangsbeatmung und der weiteren Aufnahme standhalten kann.

Weiterhin umfasst die Adapteranordnung b) einen an die weitere Aufnahme ankoppelbaren Adapteraufsatz, wobei der Adapteraufsatz über eine Dichtung gasdicht mit dem Adaptergrundkörper verbindbar und über Haltemittel an diesem mechanisch fixierbar ausgestaltet ist. An die weitere Aufnahme der Adapteranordnung lässt sich also ein Adapteraufsatz gasdicht anbringen, welcher beispielsweise über eine Steckverbindung mechanisch an die weitere Aufnahme der Adapteranordnung gekoppelt werden kann. Die gasdichte Verbindung kann auch über eine Kupplungsverbindung hergestellt werden. Durch die Verbindbarkeit des Adapteraufsatzes kann gewährleistet werden, dass die weiteren Teile der Adapteranordnung nur in den Fällen an das System gekoppelt werden, in denen diese wirklich benötigt werden. Falls eine reine Beatmung des Patienten ohne weitere Untersuchungen gewünscht ist, kann die weitere Aufnahme über einen einfachen mechanischen Verschluss gegenüber der Umgebung hygienisch sicher abgedichtet sein. Der Adapteraufsatz kann beispielsweise eine zylindrische oder konische Symmetrie und eine Ausdehnung entlang seiner Symmetrieachse von beispielsweise 1 cm bis zu 10 cm aufweisen.

Zusätzlich umfasst die Adapteranordnung c) eine mit einem Ende am Adapteraufsatz gasdicht angeordnete elastische, schlauchförmige Hülle. Der Adapteraufsatz ist an der einen Seite mit der Adapteranordnung verbindbar und ist an seinem anderen Ende mit einer Hülle verbunden. Dabei kann die Hülle beispielsweise von außen mit dem Adapteraufsatz verbunden sein. Die Hülle kann beispielsweise den gesamten Adapteraufsatz umfassen und von außen mit diesem verklebt oder verklemmt sein. Es ist aber auch möglich, dass die schlauchförmige Hülle am Innenrand des Adapteraufsatz angebracht oder befestigt ist. Wichtig ist, dass die schlauchförmige Hülle gasdicht mit dem Adapteraufsatz verbunden ist. Eine schlauchförmige Hülle stellt einen mechanisch flexiblen und beweglichen Aufsatz dar, welcher beispielsweise durch einen dünnen Plastikschlauch oder eine -Hülle realisiert werden kann. Die Hülle ist in den Fällen elastisch, in denen die Hülle reversibel gestaucht und wieder getreckt werden kann. Beispielsweise ergibt sich eine elastische Hülle in den Fällen, in denen die Hülle mehrmals auf 1/10 ihrer ursprünglichen Länge zusammengedrückt und wieder auseinandergezogen werden kann. Bevorzugt kann die elastische Hülle aus PE oder PVC bestehen oder diese Polymere umfassen. Die elastische Hülle kann bevorzugt einen Durchmesser von größer oder gleich 0,5 cm und kleiner oder gleich 5 cm, des Weiteren bevorzugt von größer oder gleich 1,0 cm und kleiner oder gleich 2,5 cm aufweisen. Die elastische Hülle kann bevorzugt eine Längsausdehnung von größer oder gleich 15 cm und kleiner oder gleich 100 cm, des Weiteren bevorzugt von größer oder gleich 50 cm und kleiner oder gleich 90 cm aufweisen. Die elastische Hülle kann beispielsweise bei Nichtbenutzung zum Lagern in zusammengestauchter Form am Adapteraufsatz befestigbar sein.

Schließlich umfasst die Adapteranordnung d) einem mit dem anderen Ende der elastischen, schlauchförmigen Hülle gasdicht verbundenen Aufsatz, wobei der Aufsatz einen Durchgang zur schlauchförmigen Hülle mit einer darin angeordneten Dichtvorrichtung umfasst, wobei die Dichtvorrichtung dazu eingerichtet ist zylindrisch ausgestaltete Vorrichtungen aufzunehmen und diese durch den Aufsatz, die schlauchförmige Hülle, den Adapteraufsatz, den Adaptergrundkörper und den Endotrachealtubus verschiebbar und gasdicht zu führen. Am anderen Ende der schlauchförmigen Hülle ist ein weiterer Aufsatz angebracht, welcher beispielsweise über eine Klebung oder aber eine Klemmung mit der schlauchförmigen Hülle verbunden sein kann. Dieser Aufsatz weist einen Durchgang zum Inneren der schlauchförmigen Hülle auf, wobei im Durchgang mindestens ein weiteres Dichtmittel angebracht ist. Bei dem Dichtmittel dieser Dichtvorrichtung kann es sich beispielsweise um einen O-Ring handeln. Es ist aber auch möglich, dass die Dichtung über eine Klappenanordnung realisiert wird. Die Klappenanordnung kann sich bei Beaufschlagung mit einer mechanischen Kraft, beispielsweise durch Einschieben eines Bronchoskops, öffnen und die Passage des Bronchoskops durch die Dichtvorrichtung des Aufsatzes in das Hülleninnere ermöglichen. Das Bronchoskop kann insofern gasdicht gegenüber der Umgebung durch die Dichtvorrichtung in Richtung Untersuchungsort geschoben werden. Durch die Dichtvorrichtung als letzte Dichtung gegenüber der Umgebung wird ein ungewolltes Austreten potenziell infektiöser Partikel oder Aerosole verhindert. Die infektiösen Partikel oder Aerosole werden als spätestens in der schlauchartigen Hülle aufgehalten. Der Aufsatz kann des Weiteren Mittel aufweisen, um den Aufsatz reversibel an dem Adapteraufsatz oder an der Adapteranordnung zu fixieren. Der Aufsatz kann beispielsweise an diesen Komponenten durch eine magnetische Wechselwirkung gehalten werden. Demzufolge kann bei Bedarf der Aufsatz mit der Hülle angekoppelt und die Untersuchung und/oder Behandlung durch Einschieben der Untersuchungsgeräte durch die Dichtung des Aufsatzes und durch die schlauchförmige Hülle hindurch gestartet werden. Der Aufsatz kann beispielsweise zylindrisch ausgestaltet sein, wobei der Innendurchmesser des Zylinders beispielsweise größer oder gleich 0,5 cm und kleiner oder gleich 5 cm, des Weiteren bevorzugt größer oder gleich 1 cm und kleiner oder gleich 3,0 cm betragen kann.

In einer bevorzugten Ausführungsform des Intubationssystems kann die Absperrvorrichtung einen rotierbaren Verschlussmechanismus aus einem im Wesentlichen zylindrisch ausgestalteten Grundkörper mit einem im Wesentlichen mittig angeordneten Durchgang umfassten, wobei in dem Durchgang mindestens zwei entlang der Symmetrieachse voneinander beabstandete Dichtmittel angeordnet sein können. Zur sicheren mechanischen Führung und zum verbesserten Handling eines Bronchoskops oder einer Absaugvorrichtung hat sich der Einsatz zweier Dichtmittel innerhalb der Öffnung des zylindrisch ausgestalteten Grundkörpers der Absperrvorrichtung als besonders geeignet herausgestellt. Innerhalb dieses kritischen Abschnitts der Adapteranordnung wird dadurch der eingeführte Untersuchungsgegenstand besonders sicher mechanisch gehalten. Zudem trägt die doppelte Dichtungsausführung zu einer verbesserten Abdichtung gegenüber der weiteren, patientenfernen Adapteranordnung bei. Der Einsatz einer doppelten Dichtung an dieser Stelle ist nicht naheliegend, da der Fachmann annehmen müsste, dass eine doppelte Dichtung der Bewegungsfreiheit des Bronchoskopes entgegenstehen und damit kritische Handlingseigenschaften zu stark beeinträchtigt würden. Zudem könnte man annehmen, dass das Ausschleusen biologischer Proben aus der Lunge erschwert wird, da der Durchgang durch die Dichtungen nun zwei Verengungen aufweist. Beides ist überraschenderweise nicht der Fall und so verbleibt die verbesserte Dichtwirkung zwischen geschlossenem Atemluftkreislauf und der Aufnahme für die Untersuchungsvorrichtung. Die beiden Dichtungen können beispielsweise in Form von O-Ringen ausgestaltet werden.

Innerhalb einer weiter bevorzugten Ausgestaltung des Intubationssystems kann der zylindrisch ausgestaltete Grundkörper des rotierbaren Verschlussmechanismusses eine im mittig angeordneten Durchgang konusförmige Aussparung in Richtung der weiteren Aufnahme umfassen, wobei der Konuswinkel größer oder gleich 20° und kleiner oder gleich 90° betragen kann. Das Anbringen einer konusförmigen Aussparung am Verschlussmechanismus kann die Handhabungseigenschaften, beispielsweise von Bronchoskopen zur Untersuchung der Lunge, deutlich erhöhen. Zum einen kann das Ein- und Durchführen des Bronchoskops durch die Öffnung deutlich erleichtert werden. Zum anderen kann der Konus die seitliche Beweglichkeit des Bronchoskopes erweitern, welches zu einer besseren Manövrierfähigkeit zylindrischer Absaugvorrichtungen oder von Bronchoskopen beiträgt. Diese verbesserten Eigenschaften ergeben sich insbesondere in Verbindung mit einer doppelten Dichtung innerhalb der Öffnung, wobei die doppelte Dichtung insbesondere auch zu einer verbesserten Abdichtung auch bei höheren Druckdifferenzen oder schnellen Druckwechseln beitragen kann. In einer weiter bevorzugten Ausgestaltung kann der Konuswinkel größer oder gleich 30° und kleiner oder gleich 85°, des Weiteren bevorzugt größer oder gleich 45° und kleiner oder gleich 75° betragen.

Innerhalb eines weiter bevorzugten Aspektes des Intubationssystem kann die Absperrvorrichtung im Adaptergrundkörper mindestens ein Feststellmittel umfassen, wobei das Feststellmittel dazu eingerichtet sein kann die Absperrvorrichtung in einer geschlossenen Stellung zu halten. Für die Sicherheit des geschlossenen Beatmungskreislaufs hat es sich als vorteilhaft erwiesen, dass die Absperrvorrichtung der Adapteranordnung erst einmal in einer geschlossenen Stellung gehalten wird. In dieser Ausrichtung ist kein Luftaustausch der weiteren Aufnahme mit den Bestandteilen des Beatmungskreislaufes möglich. In dieser Stellung kann ein Untersuchungsinstrument nicht in den Endotrachealtubus eingeschoben werden. Das Feststellmittel kann beispielsweise eine Feder oder ein anderes mechanisches Feststellmittel sein, welches eine versehentliche Öffnung der Absperrvorrichtung ohne signifikanten und willentlichen Kraftaufwand verhindert. Erst nach Aufwendung einer minimalen Kraft kann die Federkraft überwunden und der Zugang zum Einschub des Untersuchungsinstrumentes in den Endotrachealtubus freigegeben werden. In der Offen-Stellung kann dann beispielsweise ein Bronchoskop in den Endotrachealtubus eingeschoben werden. Diese Ausgestaltung kann auch in hektischen Situation ein versehentliches Öffnen des Beatmungskreises verhindern. Zusätzlich zur Feder kann auch noch eine Feder-Arretierung angebracht sein, welche beispielsweise die Feder mechanisch in einer entsprechenden Nut halten kann. Vorteilhafterweise kann am Design der Absperrvorrichtung aktiv erkennbar sein, ob die Öffnung geschlossen oder offen ist. Die Absperrvorrichtung kann zudem aus einem durchsichtigen Material ausgestaltet sein, welches neben der optischen Kontrolle der Offen/Zu-Position auch eine Kontrolle des Arbeitsganges bezüglich möglicher Blockaden ermöglicht.

Nach einer bevorzugten Charakteristik des Intubationssystems kann die Absperrvorrichtung im Adaptergrundkörper mindestens ein Detektionsmittel zur Detektion eines Gegenstandes im Inneren des Durchgangs der Absperrvorrichtung umfassen. Zur Erhöhung der Anwendungssicherheit hat es sich als besonders vorteilhaft herausgestellt, dass die Absperrvorrichtung im Adaptergrundkörper einen Detektor aufweist, welcher einen Gegenstand im Durchgang der Absperrvorrichtung detektieren kann. Das Detektionsmittel kann beispielsweise ein einfacher mechanischer Schalter sein, welcher bei mechanischem Kontakt, beispielsweise mit einem Bronchoskop, auslöst. Das Detektionsmittel kann aber auch elektrischer oder optischer Art sein, wobei in diesen Fällen das elektrische oder optische Signal ebenfalls das Vorliegen eines Gegenstandes im Durchgang der Absperrvorrichtung anzeigt. Im Falle eines Auslösens des Detektionsmittels kann beispielsweise ein Schließen der Absperrvorrichtung gesperrt werden. Zudem kann das Detektionsmittel den frühestmöglichen Zeitpunkt anzeigen, an denen die Absperrvorrichtung gefahrlos geschlossen werden kann. Dies kann ein ungewolltes Verklemmen biologischen Materials in der Öffnung der Absperrvorrichtung verhindern. Zudem kann durch das Detektionsmittel sichergestellt werden, dass die Absperrvorrichtung auf jeden Fall sicher schließt.

In einer weiter bevorzugten Ausführungsform des Intubationssystems kann der Anschluss der elastischen Hülle am Adapteraufsatz als auch der Anschluss der elastischen Hülle am Aufsatz jeweils einzeln drehbar ausgestaltet sein. Zur besonders einfachen Handhabung einer Absaugvorrichtung oder eines Bronchoskops kann es hilfreich sein, dass insbesondere die Stellen des Intubationssystems, an denen eine enge Abdichtung zum eingeführten Gegenstand wichtig ist, drehbar ausgestaltet sind. Dies kann beispielsweise die Handhabung eines Absaugkatheter erleichtern, da an diesen Stellen keine festen Relationen zwischen Intubationssystem und Katheter hergestellt wird. Es ergeben sich größere mechanische Freiheitsgrade und insbesondere wird ein ungewolltes Verdrehen der elastischen Hülle verhindert. Des Weiteren werden die mechanischen Kräfte, welche auf die Adapteranordnung wirken verringert. Über die Verringerung der mechanischen Kräfte kann zudem der Operateur den Absaugkatheter oder das Bronchoskop präziser und einfacher manipulieren.

Innerhalb eines bevorzugten Aspektes des Intubationssystems kann zumindest ein Teil des Adaptergrundkörpers des Adaptergrundkörpers aus einem durchsichtigen Material ausgestaltet sein. Zur besseren Kontrolle der Handhabung und für eine einfache erste Begutachtung entnommenen biologischen Materials, hat sich als besonders geeignet herausgestellt, dass der Adaptergrundkörper zumindest zum Teil aus einem optisch durchsichtigen Material besteht. Geeignete Materialien können beispielsweise aus der Gruppe der Kunststoffe ausgesucht sein, wobei dem Fachmann mechanisch ausreichend feste Materialien aus dieser Gruppe bekannt sind. In dieser Ausgestaltung kann entweder der gesamte Adaptergrundkörper oder aber auch nur ein Teilbereich, beispielsweise eine Hälfte, aus dem durchsichtigen Material bestehen oder dieses aufweisen.

In einer bevorzugten Ausführungsform des Intubationssystems kann der Adaptergrundkörper Mittel zur Detektion des anliegenden Druckes und Mittel zur Steuerung der Absperrvorrichtung aufweisen, wobei das Öffnen oder Schließen der Absperrvorrichtung als Funktion des anliegenden Druckes steuerbar sein kann. Zur Optimierung des Öffnungszeitpunkt der Absperrvorrichtung hatte sich als vorteilhaft herausgestellt, dass der Adaptergrundkörper dazu ausgerüstet ist, den aktuell anliegenden Druck der Beatmungsvorrichtung zu erfassen und als Funktion des anliegenden Druckes eine Steuerung der Absperrvorrichtung ermöglicht. So ist es beispielsweise denkbar, dass bei gerade anliegenden Druckspitzen die Absperrvorrichtung nicht geöffnet werden kann. Die Absperrvorrichtung kann beispielsweise erst dann freigegeben werden, wenn im Rahmen des Beatmungszyklus ein aktuell niedriger Druck anliegt. Dies kann den Druckverlust beim Öffnen der Absperrvorrichtung reduzieren und insbesondere auch dazu beitragen, dass im Moment der Öffnung der Absperrvorrichtung eine möglichst geringe Menge an Aerosolen oder Tröpfchen durch die Öffnung der Absperrvorrichtung entweichen kann. Bei den geeigneten Mitteln kann es sich beispielsweise um einen elektronischen Drucksensor mit integrierter oder externer, elektronischer Steuerung der Absperrvorrichtung handeln. Es ist prinzipiell aber auch möglich, dass das Sperren der Absperrvorrichtung über einen mechanischen Schalter erfolgt, welcher sich als Funktion des gerade anliegenden Druckzyklus in der Adapteranordnung bewegt und erst bei niedrigeren Drücken die Freigabe der Absperrvorrichtung erlaubt. In besonders vorteilhaften Fällen kann die Adapteranordnung auch elektrisch an die Beatmungsvorrichtung gekoppelt sein. Neben dieser grundsätzlichen Steuerung können weitere mechanische oder elektrische Mittel vorhanden sein, welche in Notfällen diese grundsätzliche druckabhängige Steuerung aushebeln können. In diesen Fällen kann der Operateur im Notfall sofort und ohne Rücksicht auf die aktuelle Stufe im Beatmungszyklus eingreifen.

In einer weiteren Ausgestaltung des Intubationssystems kann an dem Adaptergrundkörper über ein mechanisches Verbindungsmittel eine Verschlusskappe mit Dichtvorrichtung angeordnet sein, wobei die Verschlusskappe dazu eingerichtet sein kann die weitere Aufnahme des Adaptergrundkörpers bei nicht angekoppelten Adapteraufsatz mechanisch zu verschließen. Im klinischen Alltag hat es sich als besonders vorteilhaft herausgestellt, dass die weitere Aufnahme prinzipiell erst einmal nur über einer weiteren Verschlusskappe, welche keinen Einschub eines Untersuchungsinstrumentes erlaubt, mit entsprechender Dichtvorrichtung an dem Adaptergrundkörper angeordnet ist. Der eigentlich Adapteraufsatz mit Einschubmöglichkeit wird in diesen Fällen erst direkt vor der anstehenden Untersuchung und Öffnung der Absperrvorrichtung aufgesetzt. Durch die Verschlusskappe mit Dichtvorrichtung ist der Adaptergrundkörper hygienisch geschützt. Ein geeignetes mechanisches Verbindungsmittel für die Verbindung zwischen der Verschlusskappe und dem Adaptergrundkörper ist beispielsweise eine Schnur oder ein Polymerstreifen, welche Verschlusskappe und Adaptergrundkörper beweglich miteinander verbinden. Nach Untersuchungsende, beispielsweise nach Entfernung der Absaugvorrichtung, kann die Verschlusskappe mit Dichtvorrichtung wieder auf dem Adaptergrundkörper angeordnet werden.

Im Rahmen einer weiterhin bevorzugten Ausgestaltung des Intubationssystems kann die elastische, schlauchförmige Hülle Mittel zur Isolierung einer biologischen Probe umfassen. Im Rahmen einer Patientenuntersuchung mittels eines Bronchoskops oder aber auch im Rahmen einer Absaugung, kann es zur klinischen Routine gehören, dass biologische Proben aus dem Lungenraum entnommen werden. Diese Proben müssen zurzeit noch umständlich gehandhabt werden, wobei in der Regel die biologischen Proben kurzzeitig der Umgebungsluft ausgesetzt werden. Um diese Kontaminationsquelle auszuschließen hat sich als günstig erwiesen, dass die schlauchartige Hülle nach Ende der Untersuchung als Probenbehälter verwendet werden kann. Dazu kann gegebenenfalls die gesamte schlauchartige Hülle oder aber nur ein Teil der schlauchartigen Hülle abtrennbar ausgestaltet sein, so dass nach Einbringen der biologischen Probe aus der Lunge in die schlauchartige Hülle dieser Bereich der schlauchartigen Hülle von der Umgebung isoliert werden kann. Insofern kann die schlauchartige Hülle mindestens zwei, bevorzugt drei, des Weiteren bevorzugt vier einzelne voneinander beabstandete Elemente aufweisen, welche die schlauchartige Hülle lokal verschließen können. In dieser Ausgestaltung kann ein Verschluss dazu dienen, dass die Adapteranordnung nicht der Umgebungsluft ausgesetzt wird. Die zweite und dritte Verschlussmöglichkeiten können dazu dienen, dass die biologische Probe isoliert wird. Die vierte Verschlussmöglichkeit kann eingesetzt werden, um die schlauchartige Hülle in Richtung des Absaugkatheters oder Bronchoskops zu isolieren. Über diese vier Verschlussmöglichkeiten der schlauchartigen Hülle kann also das gesamte System vor einem unkontrollierten Kontakt mit der Umgebungsluft geschützt und zudem eine biologische Probe isoliert und separat entnommen werden. Eine Möglichkeit zur Ausgestaltung der Isolierung kann beispielsweise in der Anordnung mehrerer Clips bestehen, welche in der Lage sind, die schlauchartige Hülle an unterschiedlichen Stellen gasdicht zu verschließen. Einer oder mehrere der Clips können beispielsweise auch Schneidvorrichtung aufweisen, welche beispielsweise das Abtrennen des Mittelteils mit der biologischen Probe ermöglichen. Diese kann dann isoliert von der Umgebungsluft weitergegeben werden.

Weitere Vorteile und vorteilhafte Ausgestaltungen der erfindungsgemäßen Gegenstände werden durch die Zeichnungen veranschaulicht und in der nachfolgenden Beschreibung erläutert. Dabei ist zu beachten, dass die Zeichnungen nur beschreibenden Charakter haben und nicht dazu gedacht sind, die Erfindung einzuschränken.

Es zeigen die:
- Fig. 1: schematisch den Aufbau des erfindungsgemäßen geschlossenen Intubationssystems;
- Fig. 2: schematisch den Aufbau der erfindungsgemäßen Adapteranordnung;
- Fig. 3: schematisch den Aufbau des erfindungsgemäßen Adaptergrundkörpers mit ankoppelbaren Adapteraufsatz;
- Fig. 4: schematisch den Aufbau des erfindungsgemäßen Adaptergrundkörpers;
- Fig. 5: schematisch den Aufbau des erfindungsgemäß ankoppelbaren Adapteraufsatz mit elastischer, schlauchförmiger Hülle und gasdicht verbundenem Aufsatz.

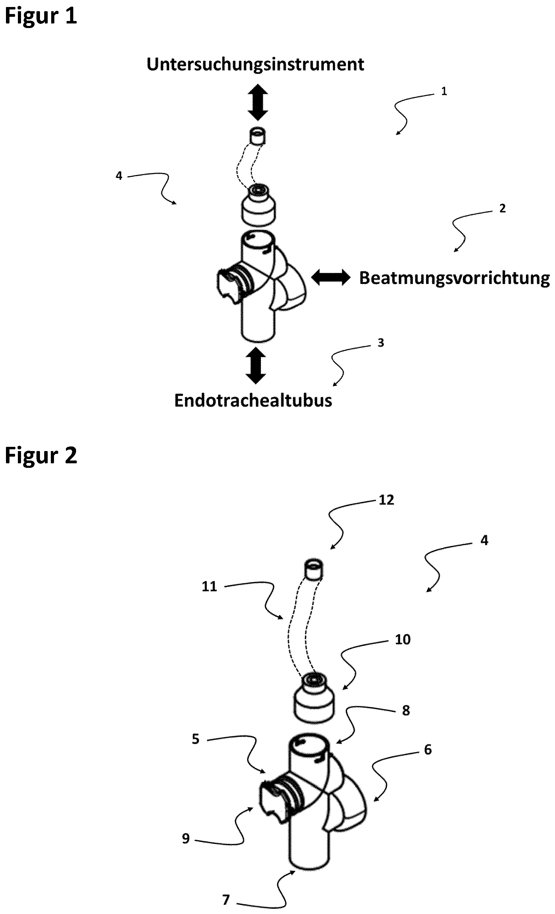

Die Figur 1 zeigt den Aufbau des erfindungsgemäßen geschlossenen Intubationssystems 1. Das geschlossene Intubationssystem 1 umfasst eine Anbindung an eine Beatmungsvorrichtung 2, einen Endotrachealtubus 3 und die erfindungsgemäße Adapteranordnung 4. Die Beatmungsvorrichtung 2 ist dazu eingerichtet, einen Patienten mit gegebenenfalls temperierter und befeuchteter Atemluft und insbesondere Sauerstoff zu versorgen. Dazu wird Luft oder eine spezifisch zusammengesetzte Luftmischung durch das geschlossene Intubationssystems 1 über einen geregelten Druckzyklus in den Patienten geleitet. Die Anbindung der Beatmungsvorrichtung 2 an die erfindungsgemäße Adapteranordnung 4 erfolgt zweckmäßigerweise über einen oder mehrere Schläuche, wobei der Schlauch der Beatmungsvorrichtung 2 an einer Aufnahme der Adapteranordnung 4 angeschlossen wird. An der zweiten Aufnahme der Adapteranordnung 4 kann ein Endotrachealtubus 3 angeschlossen werden, wobei der Tubus zur Beatmung des Patienten in dessen Lunge eingeführt werden kann. Die Adapteranordnung 4 weist zudem eine weitere Aufnahme auf, an welche über die Adapteranordnung 4 ein Untersuchungsinstrument angeschlossen werden kann. Das Untersuchungsinstrument kann beispielsweise ein Bronchoskop oder eine Absaugeinrichtung sein, wobei die Untersuchungsinstrumente dazu ausgelegt sind, zumindest teilweise, in die Lunge des Patienten eingeführt zu werden. Das Untersuchungsinstrument wird über die Aufnahme der Adapteranordnung 4, durch den Endotrachealtubus 3 in die Lunge des Patienten eingeführt. Insbesondere kann durch die Ausgestaltung des Adapteraufsatzes 10 der Adapteranordnung 4 das Untersuchungsinstrument flexibel und sicher geführt werden, wobei insbesondere keine möglicherweise kontaminierten Aerosole, Partikel oder Tröpfchen während der Untersuchung in die Umgebung gelangen können. Durch dieses geschlossene Intubationssystem 1 wird das medizinische Personal im Fall einer Untersuchung vor einer Infektion besonders effizient geschützt. Das geschlossene Intubationssystem 1 ist flexibel einsetzbar und kann auch unter hektischen Untersuchungsbedingungen sicher gehandhabt werden.

Die Figur 2 zeigt schematisch den Aufbau der erfindungsgemäßen Adapteranordnung 4 des geschlossenen Intubationssystems 1. Die Adapteranordnung 4 umfasst einen Adaptergrundkörper 5, welcher über die drei Aufnahmen zum Tubus 3, zur Beatmungsvorrichtung 2 und zum Untersuchungsinstrument T-förmig aufgebaut ist. Dementsprechend weist die Adapteranordnung 4 einen Aufnahme 6 für die Anbindung zur Beatmungsvorrichtung 2 und eine Aufnahme 7 zur Anbindung an den Endotrachealtubus 3 auf. Diese beiden Aufnahmen 6, 7 sind über eine Absperrvorrichtung 9 gegenüber der weiteren Aufnahme 8 gasdicht verschließbar. Auf dem Adaptergrundkörper 5 kann auf der weiteren Aufnahme 8 im Falle einer durchzuführenden Untersuchung ein Adapteraufsatz 10 angeordnet werden. Diese Aufnahme 8 kann aber in den Fällen, in denen keine Untersuchung durchgeführt wird, mit einer Kappe mit Dichtvorrichtung verschlossen sein. Der Adapteraufsatz 10 kann also nur kurz vor der Durchführung einer Untersuchung auf den Adaptergrundkörper 5 aufgesteckt und mechanisch fixiert werden. In dieser Figur sind hierzu Nut/Feder-Mittel aufgezeigt, welche zur mechanischen Fixierung zwischen Adaptergrundkörper 5 und Adapteraufsatz 10 dienen können. Der Adapteraufsatz 10 ist zudem über eine innenliegende Dichtung gasdicht mit dem Adaptergrundkörper 5 verbindbar. An dem Adapteraufsatz 10 ist eine schlauchartige Hülle 11 angeordnet und mit diesem gasdicht verbunden. Die schlauchartige Hülle 11 dient zur flexiblen Aufnahme des vorderen Teils des Untersuchungsinstrumentes und kann zusätzlich auch noch biologische Proben des Patienten aufnehmen. Die schlauchartige Hülle 11 verhindert beim Einführen des Untersuchungsinstrumentes, dass Aerosole oder Partikel aus dem geschlossenen Intubationssystem 1 in die Umgebung abgegeben werden können. Die Aerosole können zwar in den Bereich des Adaptergrundkörpers 5 und eventuell noch durch den Adapteraufsatz 10 austreten, werden aber durch die schlauchartige Hülle 11 und den daran befestigten Aufsatz 12 in Verbindung mit dem Untersuchungsinstrument sicher eingeschlossen. Eine derartige Isolierung gegenüber Aerosolen oder Partikeln ist dadurch nicht erreichbar, dass nur das Untersuchungsinstrument als solches in einer verschiebbaren gekapselt vorliegt. Wichtig ist die Isolierung von Aerosolen in einem Bereich, in welchem das Untersuchungsinstrument noch die Umgebung gegenüber dem geschlossenen Intubationssystems 1 isoliert. Dazu ist der Aufsatz 12 über eine Dichtung mit dem Untersuchungsinstrument gasdicht verbunden. Das Einführen des vorderen Teils des Untersuchungsinstrumentes über den Aufsatz 12, die schlauchartige Hülle 11 und den Adapteraufsatz 10 in das an sich geschlossene Intubationssystem 1, erfolgt über die Absperrvorrichtung 9. Die Absperrvorrichtung 9 kann beispielsweise drehbar ausgestaltet sein und weist eine Öffnung zur Durchführung des Untersuchungsinstrumentes auf. Nach dem Öffnen der Absperrvorrichtung 9 kann das Untersuchungsinstrument durch die Aufnahme 7 in den daran angeordneten Endotrachealtubus 3 eingeführt werden.

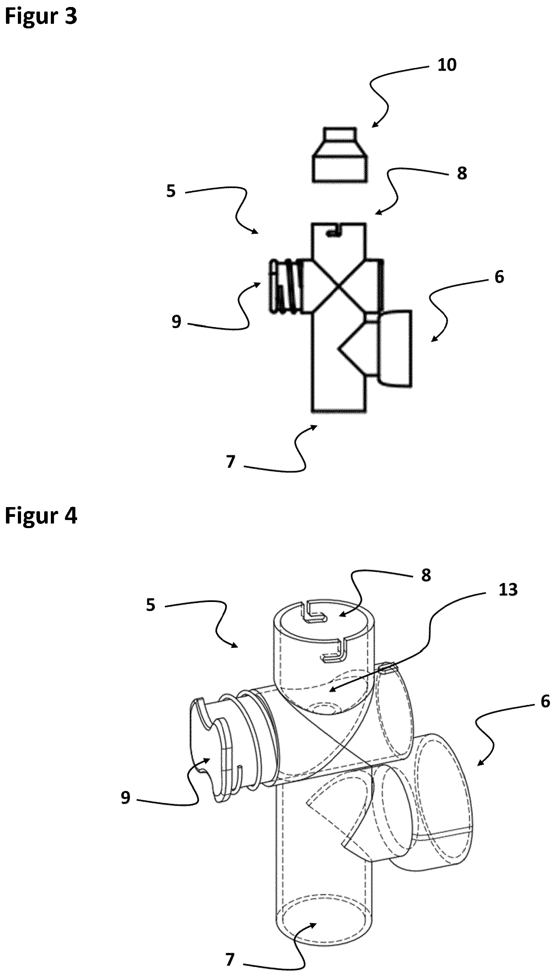

Die Figur 3 zeigt schematisch den Aufbau des erfindungsgemäßen Adaptergrundkörpers 5 mit ankoppelbaren Adapteraufsatz 10. Der Adaptergrundkörper 5 weist eine Aufnahme 6 zur Beatmungsvorrichtung und eine Aufnahme 7 zum Endotrachealtubus 3 auf. Diese beiden Aufnahmen sind durch die Absperrvorrichtung 9 gegenüber der Aufnahme 8 zum Untersuchungsinstrument hin absperrbar. Des Weiteren zeigt die Figur den Adapteraufsatz 10, welcher über eine innenliegende Dichtvorrichtung gasdicht mit dem Adaptergrundkörper 5 verbindbar ausgestaltet ist. Die Absperrvorrichtung 9 weist innenliegend (nicht dargestellt) eine oder mehrere Dichtungen auf, durch welche das Untersuchungsinstrument im Falle einer durchzuführenden Untersuchung geschoben werden kann. Die Absperrvorrichtung 9 kann noch ein Arretierungsmittel aufweisen, in welcher die Absperrvorrichtung 9 in offener Position arretiert wird (nicht dargestellt). Weiterhin kann die Absperrvorrichtung 9, beispielsweise durch eine Feder, in geschlossener Stellung gehalten werden, wobei zum Öffnen der Absperrvorrichtung 9 erst die Federkraft überwunden werden muss. Dies kann ein zu leichtes oder versehentliches Öffnen der Absperrvorrichtung 9 verhindern.

Die Figur 4 zeigt schematisch den Aufbau des erfindungsgemäßen Adaptergrundkörpers mit der Aufnahme 6 zur Beatmungsvorrichtung 2, der Aufnahme 7 zum Endotrachealtubus 3 und der weiteren Aufnahme 8, zum Anschluss des Adapteraufsatzes 10 (nicht dargestellt). Der Adaptergrundkörper 5 ist über die Absperrvorrichtung 9 unterteilbar. Die Absperrvorrichtung 9 ist in diesem Fall zylindrisch ausgestaltet und weist einen mittig angeordneten Durchgang mit zwei innenliegenden Dichtvorrichtungen beispielsweise in Form von O-Ringen oder Klappensegeln auf. Durch die Dichtungen kann ein zylindrisches Untersuchungsinstrument gasdicht geführt oder geschoben werden. In dieser Figur ist zu erkennen, dass der mittig angeordnete Durchgang 13 eine konusförmige Aussparung in Richtung der weiteren Aufnahme aufweist. Der Konuswinkel beträgt in diesem Fall ca. 20-40°. Durch die Ausbildung einer konusförmigen Vertiefung kann die Handhabung und insbesondere das ansatzlose Einführen des Untersuchungsinstrumentes in den Tubus deutlich erleichtert werden. Zudem kann über diese Ausgestaltung ein schnelleres und gezielteres Einführen des Untersuchungsinstrumentes auch unter Zeitdruck erreicht werden. Ohne durch die Theorie gebunden zu sein ergibt sich das wahrscheinlich dadurch, dass die untere Form von Absaugvorrichtungen oder Bronchoskopen durch ihre konische Ausgestaltung leichter zur eigentlichen Öffnung geführt werden können. Dies kann sich beispielsweise auch schon bei nicht vollständig geöffneter Absperrvorrichtung 9 ergeben, welches dann entsprechend eine zusätzliche Kraft durch das Untersuchungsinstrument zum Öffnen der Absperrvorrichtung 9 hervorruft. Die Öffnung wird zudem effizienter durch das Untersuchungsinstrument abgedeckt, welches die Ausbreitung von Aerosolen oder Tröpfchen aus der Lunge des Patienten in Richtung weiterer Aufnahme 8 verringert.

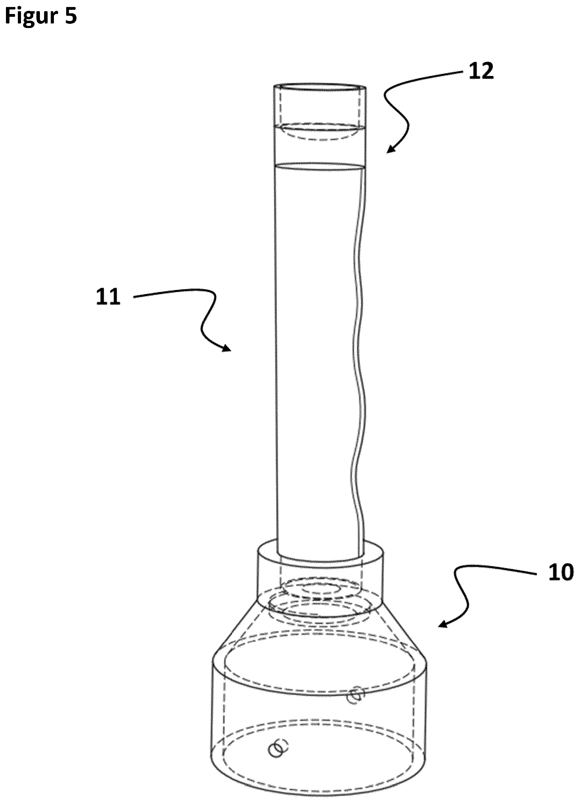

Die Figur 5 zeigt schematisch den Aufbau des erfindungsgemäß ankoppelbaren Adapteraufsatz 10 mit elastischer, schlauchförmiger Hülle 11 und gasdicht verbundenem Aufsatz 12. Die schlauchartige Hülle 11 kann beispielsweise einen Durchmesser von um größer oder gleich 1,5 cm und kleiner oder gleich 5 cm und eine Länge von um größer oder gleich 10 cm und kleiner oder gleich 100 cm aufweisen. Die schlauchartige Hülle 11 ist sowohl mit dem Adapteraufsatz 10 und dem Aufsatz 12 über eine Klemmung oder eine Klebung gasdicht verbunden. Die schlauchartige Hülle 11 ist zusammenschieb- und ausziehbar ausgerüstet, sodass die Länge der schlauchartigen Hülle 11 im Rahmen der Untersuchung verändert werden kann. Die schlauchartige Hülle 11 kann zudem Vorrichtungen aufweisen, um in mehrere Kompartimente aufgeteilt zu werden. Der Aufsatz 12 und der Adapteraufsatz 10 weisen jeweils in ihrem Inneren mindestens eine Dichtung auf, welche auf den Durchmesser der Untersuchungsvorrichtung angepasst oder anpassbar ist. Es können auch verschiedene Versionen mit unterschiedlichen Dichtungsdurchmesser angefertigt werden. Nach Beendigung der Untersuchung wird beispielsweise ein Bronchoskop wieder aus der Lunge des Patienten geführt, indem das Bronchoskop in Richtung Aufsatz 12 gezogen wird. Das Bronchoskop passiert den Adaptergrundkörper 5 und wird erst durch die erste dann durch die zweite Dichtung der Absperrvorrichtung 9 geführt. Eventuell freigesetzte Aerosole werden in diesem Fall im Adaptergrundkörper 5 festgehalten, da das Bronchoskop am Dichtmittel des Adapteraufsatz 10 abdichtet. Wird das Bronchoskop auch durch diese Dichtung geführt, so werden noch verbleibende Aerosole in der schlauchartigen Hülle 11 festgehalten. In dieser Stellung oder schon nach Durchführung des Instrumentes durch die Dichtungen des Adaptergrundkörpers 5 kann die Absperrvorrichtung 9 geschlossen werden. Dies reduziert die Gefahr einer weiteren Freisetzung von Aerosolen aus dem geschlossenen Intubationssystems 1. Die schlauchartige Hülle 11 kann dann nach teilweisem Durchführen des Bronchoskops selbst noch durch optionale Einschnürungsmöglichkeiten in Kompartimente aufgeteilt werden, wobei eventuell aus der Lunge entnommenes Material einer weiteren Untersuchung zugeführt werden kann. Schließlich wird das Bronchoskop durch den dichtenden Aufsatz 12 geführt. Die erfindungsgemäße Adapteranordnung kann am Aufsatz 12 durch eine weitere Kappe verschlossen und entsorgt werden.

## Patentansprüche

1. Geschlossenes Intubationssystem (1) mindestens umfassend einen für die Einführung in die Luftröhre eines Patienten geeigneten Endotrachealtubus (3), eine schlauchförmige Anbindung an eine Beatmungsvorrichtung (2) und eine Adapteranordnung (4) mit mindestens drei zylindrisch ausgestalteten Aufnahmen (6,7,8) zur Herstellung einer gasdichten Kopplung zwischen Endotrachealtubus (3), der Anbindung der Beatmungsvorrichtung (2) und mindestens einer weiteren Aufnahme (8), wobei die Adapteranordnung (4) mindestens umfasst:
a) einen über die Anordnung der Aufnahmen (6,7,8) T-förmig ausgestalteten Adaptergrundkörper (5) mit mindestens einer Absperrvorrichtung (9), wobei die Absperrvorrichtung (9) dazu eingerichtet ist den Luftweg zwischen der weiteren Aufnahme (8) und den beiden Aufnahmen für (6,7) die Beatmungsvorrichtung (2) und den Endotrachealtubus (3) gasdicht zu öffnen oder zu schließen,
b) einen an die weitere Aufnahme (8) ankoppelbaren Adapteraufsatz (10), wobei der Adapteraufsatz (10) über eine Dichtung gasdicht mit dem Adaptergrundkörper (4) verbindbar und über Haltemittel an diesem mechanisch fixierbar ausgestaltet ist;
c) eine mit einem Ende am Adapteraufsatz (10) gasdicht angeordnete elastische, schlauchförmige Hülle (11);
d) einem mit dem anderen Ende der elastischen, schlauchförmigen Hülle (11) gasdicht verbundenen Aufsatz (12), **dadurch gekennzeichnet, dass** der Aufsatz (12) einen Durchgang zur schlauchförmigen Hülle (11) mit einer darin angeordneten Dichtvorrichtung umfasst, wobei die Dichtvorrichtung dazu eingerichtet ist zylindrisch ausgestaltete Vorrichtungen aufzunehmen und diese durch den Aufsatz (12), die schlauchförmige Hülle (11), den Adapteraufsatz (10), den Adaptergrundkörper (5) und den Endotrachealtubus (3) verschiebbar und gasdicht zu führen.

2. Intubationssystem nach Anspruch 1, wobei die Absperrvorrichtung (9) einen rotierbaren Verschlussmechanismus aus einem im Wesentlichen zylindrisch ausgestalteten Grundkörper mit einem im Wesentlichen mittig angeordneten Durchgang umfasst, wobei in dem Durchgang mindestens zwei entlang der Symmetrieachse voneinander beabstandete Dichtmittel angeordnet sind.

3. Intubationssystem nach Anspruch 2, wobei der zylindrisch ausgestaltete Grundkörper des rotierbaren Verschlussmechanismusses (9) eine im mittig angeordneten Durchgang konusförmige Aussparung (13) in Richtung der weiteren Aufnahme umfasst, wobei der Konuswinkel größer oder gleich 20° und kleiner oder gleich 90° beträgt.

4. Intubationssystem nach einem der vorhergehenden Ansprüche, wobei die Absperrvorrichtung (9) im Adaptergrundkörper (5) mindestens ein Feststellmittel umfasst, wobei das Feststellmittel dazu eingerichtet ist die Absperrvorrichtung (9) in einer geschlossenen Stellung zu halten.

5. Intubationssystem nach einem der Ansprüche 2 bis 4, wobei die Absperrvorrichtung (9) im Adaptergrundkörper (5) mindestens ein Detektionsmittel zur Detektion eines Gegenstandes im Inneren des Durchgangs der Absperrvorrichtung umfasst.

6. Intubationssystem nach einem der vorhergehenden Ansprüche, wobei der Anschluss der elastischen Hülle (11) am Adapteraufsatz (10) als auch der Anschluss der elastischen Hülle (11) am Aufsatz (12) jeweils einzeln drehbar ausgestaltet sind.

7. Intubationssystem nach einem der vorhergehenden Ansprüche, wobei zumindest ein Teil des Adaptergrundkörpers (5) aus einem durchsichtigen Material ausgestaltet ist.

8. Intubationssystem nach einem der vorhergehenden Ansprüche, wobei der Adaptergrundkörper (5) Mittel zur Detektion des anliegenden Druckes und Mittel zur Steuerung der Absperrvorrichtung (9) aufweist, wobei das Öffnen oder Schließen der Absperrvorrichtung (9) als Funktion des anliegenden Druckes steuerbar ist.

9. Intubationssystem nach einem der vorhergehenden Ansprüche, wobei an dem Adaptergrundkörper (5) über ein mechanisches Verbindungsmittel eine Verschlusskappe mit Dichtvorrichtung angeordnet ist, wobei die Verschlusskappe dazu eingerichtet ist die weitere Aufnahme (8) des Adaptergrundkörpers bei nicht angekoppelten Adapteraufsatz (10) mechanisch zu verschließen.

10. Intubationssystem nach einem der vorhergehenden Ansprüche, wobei die elastische, schlauchförmige Hülle (11) Mittel zur Isolierung einer biologischen Probe umfasst.

## Claims

1. A closed intubation system (1) comprising at least an endotracheal tube (3) suitable for insertion into the trachea of a patient, a tubular connection to a breathing device (2) and an adapter arrangement (4) with at least three cylindrically designed receptacles (6, 7, 8) for establishing a gas-tight coupling between the endotracheal tube (3), the connection of the breathing device (2) and at least one further receptacle (8), wherein the adapter arrangement (4) comprises at least:
a) an adapter base body (5) with at least one shut-off device (9), which is configured in a T-shape via the arrangement of the receptacles (6, 7, 8), the shut-off device (9) being designed to open or close the airway between the further receptacle (8) and the two receptacles (6, 7) for the breathing device (2) and the endotracheal tube (3) in a gas-tight manner,
b) an adapter attachment (10) that can be coupled to the further receptacle (8), wherein the adapter attachment (10) can be connected in a gas-tight manner to the adapter base body (4) via a seal and is designed to be mechanically fixed to the latter by holding means;
c) an elastic, tubular sheath (11) that is arranged in a gas-tight manner at one end on the adapter attachment (10);
d) an attachment (12) connected in a gas-tight manner to the other end of the elastic, tubular sheath (11), **characterised in that** the attachment (12) comprises a passage to the tubular sheath (11) with a sealing device arranged therein, wherein the sealing device is configured to receive cylindrically configured devices and to guide them in a displaceable and gas-tight manner through the attachment (12), the tubular sheath (11), the adapter attachment (10), the adapter base body (5) and the endotracheal tube (3).

2. Intubation system according to claim 1, wherein the shut-off device (9) comprises a rotatable closure mechanism consisting of a substantially cylindrical base body with a substantially centrally arranged passage, wherein at least two sealing means are arranged in the passage, spaced apart from one another along the axis of symmetry.

3. Intubation system according to claim 2, wherein the cylindrically configured base body of the rotatable shut-off mechanism (9) comprises a conical recess (13) in the centrally arranged passage in the direction of the further receptacle, wherein the cone angle is greater than or equal to 20° and less than or equal to 90°.

4. Intubation system according to any one of the preceding claims, wherein the shut-off device (9) in the adapter base body (5) comprises at least one locking means, the locking means being configured to hold the shut-off device (9) in a closed position.

5. Intubation system according to any one of claims 2 to 4, wherein the shut-off device (9) in the adapter base body (5) comprises at least one detection means for detecting an object in the interior of the passage for the shut-off device.

6. Intubation system according to any one of the preceding claims, wherein the connection of the elastic sheath (11) to the adapter attachment (10) and the connection of the elastic sheath (11) to the attachment (12) are each individually rotatable.

7. Intubation system according to any one of the preceding claims, wherein at least a part of the adapter base body (5) is made of a transparent material.

8. Intubation system according to any one of the preceding claims, wherein the adapter base body (5) has means for detecting the applied pressure and means for controlling the shut-off device (9), wherein the opening or closing of the shut-off device (9) can be controlled as a function of the applied pressure.

9. Intubation system according to any one of the preceding claims, wherein a sealing cap with a sealing device is arranged on the adapter base body (5) using a mechanical connecting means, the sealing cap being designed to mechanically seal the further receptacle (8) on the adapter base body when the adapter attachment (10) is not coupled.

10. An intubation system according to any one of the preceding claims, wherein the elastic tubular sheath (11) comprises means for isolating a biological sample.

## Revendications

1. Système d'intubation fermé (1) comprenant au moins une sonde endotrachéale (3) pouvant être insérée dans la trachée du patient, une connexion tubulaire à un dispositif respiratoire (2) et un adaptateur (4) doté d'au moins trois logements cylindriques (6, 7, 8) permettant d'établir un couplage étanche aux gaz entre le tube endotrachéale (3), la connexion du dispositif respiratoire (2) et au moins un autre logement (8), l'adaptateur (4) comprenant au moins :
a) un corps de base de l'adaptateur (5) avec au moins un dispositif d'arrêt (9) configuré en T par la disposition des logements (6, 7, 8), le dispositif d'arrêt (9) étant conçu pour ouvrir ou fermer de manière étanche au gaz la voie aérienne entre le logement supplémentaire (8) et les deux logements (6, 7) pour le dispositif respiratoire (2) et le tube endotrachéal (3) ;
b) un adaptateur (10) pouvant être couplé à l'autre logement (8), l'adaptateur (10) pouvant être relié de manière étanche au gaz au corps de base de l'adaptateur (4) par l'intermédiaire d'un joint, et conçu pour être fixé mécaniquement à ce dernier par des moyens de fixation ;
c) une gaine tubulaire élastique (11) disposée de manière étanche au gaz à une extrémité sur l'embout de l'adaptateur (10) ;
d) un embout (12) relié de manière étanche au gaz à l'autre extrémité de la gaine tubulaire élastique (11), **caractérisé en ce que** l'embout (12) comprend un passage vers la gaine tubulaire (11) avec un dispositif d'étanchéité disposé à l'intérieur, le dispositif d'étanchéité étant conçu pour recevoir des dispositifs de configuration cylindrique et pour les guider de manière déplaçable et étanche au gaz à travers l'embout (12), la gaine tubulaire (11), l'embout de l'adaptateur (10), le corps de base de l'adaptateur (5) et le tube endotrachéal (3).

2. Système d'intubation selon la revendication 1, dans lequel le dispositif d'arrêt (9) comprend un mécanisme de fermeture rotatif constitué d'un corps de base principalement cylindrique avec un passage disposé sensiblement au centre, dans lequel au moins deux dispositifs d'étanchéité sont placés dans le passage, espacés l'un de l'autre le long de l'axe de symétrie.

3. Système d'intubation selon la revendication 2, dans lequel le corps de base de configuration cylindrique du mécanisme d' arrêt rotatif (9) comprend un évidement conique (13) dans le passage placé au centre en direction du logement supplémentaire, dans lequel l'angle du cône est supérieur ou égal à 20° et inférieur ou égal à 90°.

4. Système d'intubation selon l'une des revendications précédentes, dans lequel le dispositif d'arrêt (9) dans le corps de base de l'adaptateur (5) comprend au moins un dispositif de verrouillage, le dispositif de verrouillage étant configuré pour maintenir le dispositif d'arrêt (9) dans une position fermée.

5. Système d'intubation selon l'une des revendications 2 à 4, dans lequel le dispositif d'arrêt (9) dans le corps de base de l'adaptateur (5) comprend au moins un dispositif de détection pour détecter un objet à l'intérieur du passage pour le dispositif d'arrêt.

6. Système d'intubation selon l'une des revendications précédentes, dans lequel la connexion de la gaine élastique (11) à l'adaptateur (10) et la connexion de la gaine élastique (11) à l'embout (12) sont chacune rotatives individuellement.

7. Système d'intubation selon l'une des revendications précédentes, dans lequel au moins une partie du corps de base de l'adaptateur (5) est constituée d'un matériau transparent.

8. Système d'intubation selon l'une des revendications précédentes, dans lequel le corps de base de l'adaptateur (5) comporte des dispositifs de détection de la pression appliquée et des méthodes de commande du dispositif d'arrêt (9), l'ouverture ou la fermeture du dispositif d'arrêt (9) pouvant être commandée en fonction de la pression appliquée.

9. Système d'intubation selon l'une des revendications précédentes, dans lequel un capuchon d'étanchéité avec un dispositif d'étanchéité est disposé sur le corps de base de l'adaptateur (5) à l'aide d'un dispositif de liaison mécanique, le capuchon d'étanchéité étant conçu pour sceller mécaniquement l'autre logement (8) sur le corps de base de l'adaptateur lorsque l'embout de l'adaptateur (10) n'est pas couplé.

10. Système d'intubation selon l'une des revendications précédentes, dans lequel la gaine tubulaire élastique (11) comprend des dispositifs pour isoler un échantillon biologique.
